# EUROPÄISCHE PATENTSCHRIFT

(11) **EP 2 094 667 B1**
(45) Veröffentlichungstag und Bekanntmachung des Hinweises auf die Patenterteilung: **13.01.2016**
(21) Anmeldenummer: 07722440.0
(22) Anmeldetag: 17.05.2007
(51) Int. Cl.: C07C 211/63, C07D 233/06, C07D 233/54, C07D 233/56, C07F 9/54, B01J 20/22, H01M 10/052

(54) **SALZE AUS PENTAFLUORPHENYLIMID- ANIONEN UND IHRE VERWENDUNG ALS IONISCHE FLÜSSIGKEITEN**
SALTS CONSISTING OF PENTAFLUORPHENYLIMIDE ANIONS AND THEIR USE AS IONIC LIQUIDS
SELS D'ANIONS PENTAFLUOROPHENYLIMIDE ET LEUR APPLICATION COMME LIQUIDES IONIQUES

(30) Priorität: 17.05.2006 DE 102006023649
(43) Veröffentlichungstag der Anmeldung: 02.09.2009
(73) Patentinhaber: Philipps-Universität Marburg, 35037 Marburg (DE)
(72) Erfinder: SUNDERMEYER, Jörg, 35041 Marburg (DE); LINDER, Thomas, 35039 Marburg (DE)
(74) Vertreter: Patentanwälte Olbricht Buchhold Keulertz
(86) Internationale Anmeldenummer: PCT/DE2007/000892
(87) Internationale Veröffentlichungsnummer: WO 2007/131498

(56) Entgegenhaltungen:
- EP-A1- 1 414 088
- WO-A1-2004/054991
- KHVOROST, ALEXANDER ET AL: "Lithium bis(pentafluorophenyl)amide - Syntheses and structural characterization of its complexes with diethyl ether and THF" ZEITSCHRIFT FÜR ANORGANISCHE UND ALLGEMEINE CHEMIE, Bd. 630, Nr. 6, 2004, Seiten 885-889, XP002453409
- NAGY, ATTILA ET AL: "He(I) and He(II) photoelectron spectroscopic investigation of substituent effects in aminosilanes" JOURNAL OF ORGANOMETALLIC CHEMISTRY, Bd. 419, Nr. 1-2, 1991, Seiten 27-42, XP002453410
- KOPPANG, ROLF: "Poly-N-Methyliminotetrafluoro-1,4-Phenyle ne" JOURNAL OF POLYMER SCIENCE, POLYMER CHEMISTRY EDITION, Bd. 14, Nr. 9, 1976, Seiten 2225-2231, XP002453411
- DATABASE CAPLUS [Online] CHEMICAL ABSTRACTS SERVICE, COLUMBUS, OHIO, US; 1982, VLASOV, V. M. ET AL: "Polyfluoroaryl-containing N-anions, proton and fluorine-19 NMR spectra of their salts" XP002453414 gefunden im STN Database accession no. 1982:68187 & ZHURNAL ORGANICHESKOI KHIMII , 17(10), 2192-201 CODEN: ZORKAE; ISSN: 0514-7492, 1981,
- CHIANG Y ET AL: "Flash Photolytic Generation of Primary, Secondary, and Tertiary Ynamines in Aqueous Solution and Study of Their Carbon-Protonation Reactions in That Medium" JOURNAL OF THE AMERICAN CHEMICAL SOCIETY., Bd. 118, Nr. 18, 1996, Seiten 4366-4372, XP002453450

## Beschreibung

### Hydrophobe ionische Flüssigkeiten

Die vorliegende Erfindung betrifft Salze aus Pentafluorphenylimid-Anionen und Kationen, ausgewählt aus anorganischen Kationen aus der Gruppe der Alkalikationen und Erdalkalikationen oder quaternären organischen Kationen, ein Verfahren zu ihrer Herstellung sowie ihre Verwendung als hydrophobe ionische Flüssigkeiten.

### Beschreibung und Einleitung des allgemeinen Gebietes der Erfindung

Die vorliegende Erfindung betrifft die Gebiete Elektrochemie, Materialchemie, organische Chemie und Reaktionstechnik.

### Stand der Technik

Ionische Flüssigkeiten sind Verbindungen, die aus einem organischen Kation und einem anorganischen oder organischen Anion bestehen. Sie enthalten keine neutralen Moleküle und besitzen Schmelzpunkte unter 100 °C. Im Stand der Technik sind eine Vielzahl von Verbindungen bekannt, die als ionische Flüssigkeiten Verwendung finden. Insbesondere sind sie auch Gegenstand einer Reihe von Patenten bzw. Patentanmeldungen.
Einige dieser Offenbarungen beschreiben hydrophobe ionische Flüssigkeiten, wobei häufig versucht wird, die Hydrophobie durch die Einführung von Halogenatomen oder langen Alkylketten in Anionen oder Kationen zu erhöhen. So offenbart die JP 2005 314 332 A1 ionische Flüssigkeiten, deren Anion Fluoroalkylsulfate enthalten. Die DE 103 33 239 A1 beschreibt ionische Flüssigkeiten, deren Anionen unter anderem Bis-perperfluoralkylsulfonylimide [N(SO₂Rf)₂]⁻ oder Trisperfluoralkylsulfonylmethide [C(SO₂Rf)₃]⁻ sein können. Die DE 102 58 671 A1 offenbart ionische Flüssigkeiten mit Bis-(trifluormethyl)-imidanionen.

Dem Fachmann ist bekannt, dass Dipentafluorphenylamin sehr hydrophob und wenig basisch ist. Diese Verbindung kann gemäß R Koppang, Acta Chem. Scand 1971, 3067-3071, durch Umsetzung von C₆F₆ mit einem Metallamid, bevorzugt LiNH₂ nach folgenden Reaktionsgleichungen (auch als Eintopfreaktion) hergestellt werden

LiNH₂ + C₆F₆ → LiF + H₂N-C₆F₅

H₂NC₆F₅ + LiNH₂ → LiNH-C₆F₅ + NH₃

2LiNHC₆F₅ + C₆F₆ → LiN(C₆F₅)₂ + H₂NC₆F₅ + LiF

LiN(C₆F₅)₂ + HX → HN(C₆F₅)₂ + LiX

Dabei steht X beispielsweise für ein Halogenatom, Nitrat, Hydrogensulfat oder Dihydrogenphosphat.
Im Stand der Technik existieren jedoch keine Hinweise bezüglich der Eignung von Dipentafluorphenylamin und seines korrespondierenden Anions (Dipentafluorphenylanilids bzw. Decafluordiphenylimid) zur Herstellung ionischer Flüssigkeiten.

Ionische Flüssigkeiten werden beispielsweise als Bestandteile von Elektrolytsystemen in Batterien, Akkumulatoren, galvanischen Elementen und Brennstoffzellen oder als Lösungsmittel in Synthesen verwendet.

Einige bekannte ionische Flüssigkeiten enthalten Imidazoliumkationen. Diese ionischen Flüssigkeiten sind besonders vorteilhaft als Lösungsmittel für edelmetallkatalysierte Synthesen zu verwenden. So beschreibt beispielsweise die DE 10 2004 034 543 A1 ein Verfahren zur Herstellung von Oniumsalzen mit geringem ChloridGehalt, zur Verwendung als Kationen in ionischen Flüssigkeiten. Bei diesen Kationen kann es sich u.a. um Imidazoliumkationen handeln, darunter auch um 2H-Imidazoliumkationen. Des Weiteren beschreibt die WO 01/77081 A1 ein Verfahren zur Herstellung von 2H-Imidazolium-Salz-basierten ionische Flüssigkeiten über die Umsetzung einer Säure oder eines Alkohols mit nukleophilen N-heterocyclischen Carben-Vorstufen. Ionische Flüssigkeiten, die auf 2H-Imidazoliumkationen basieren, sind einerseits sehr gute Lösungsmittel für edelmetallkatalysierte Synthesen. Andererseits haben sie den Nachteil, dass es vor allem in Gegenwart von Platinkatalysatoren leicht zur einer C-H-Aktivierung am C2-Atom des Imidazoliumkations unter oxidativer Addition an das Edelmetallzentrum und Bildung eines Carbenkomplexes kommt. Dies führt häufig zu einem unerwünschten Aktivitätsverlust des Katalysators. 2-Alkyl-imidazolium-basierte ionische Flüssigkeiten sind in dieser Hinsicht stabiler.

Die Eigenschaften ionischer Flüssigkeiten, z.B. Schmelzpunkt, thermische und elektrochemische Stabilität und Viskosität, werden stark von der Natur des Anions beeinflusst. Demgegenüber können die Polarität und die Hydrophilie bzw. Lipophilie durch geeignete Wahl des Kation/Anion-Paares variiert werden.

Beim Einsatz ionischer Flüssigkeiten ist deren Reinheit von großer Bedeutung. Verunreinigungen in ionischen Flüssigkeiten können beispielsweise den Verlauf chemischer Reaktionen negativ beeinflussen. Daher besteht Bedarf an Verfahren, die eine Einführung eines Anions durch quantitative chemische Reaktion erlauben und nicht durch Gleichgewichtsverschiebung beim Ionenaustausch. Weiterhin besteht großer Bedarf an neuen äußerst hydrophoben / lipophilen ionischen Flüssigkeiten am Ende der Polaritätsskala, die neue Möglichkeiten hinsichtlich ihrer Verwendung in der Mehrphasenkatalyse (Nicht-Mischbarkeit mit Wasser) oder in elektrochemischen und analytischen Anwendungen bieten.

Die EP 1 414 088 A1 betrifft verschiedene Salze enthaltend N-Pentafluorophenylsubstituierte Carbonsäureamid-Anionen sowie deren Verwendung als nicht wässriger Elektrolyt. Diese Verbindungen sind durch einen am am Stickstoff vorgesehenen C=O-Substituenten gekennzeichnet. Die in EP 1 414 088 A1 beschriebenen Carbonsäureamid-Salze weisen unter anderem eine Stickstoff-gebundene Pentafluorophenylgruppe auf. Derartige Salze können jedoch nicht mit hoher Reinheit und hoher Ausbeute hergestellt werden. Die in EP 1 414 088 A1 beschriebenen Verbindungen sind durch Ionen-Austauschreaktionen herzustellen. Hierbei sind geringe Mengen an Verunreinigung durch das als Nebenprodukt entstehende Salz, wie Na-Salz, und/oder Edukt, nicht zu vermeiden. Derartige Verunreinigungen können den Verlauf von chemischen Reaktionen negativ beeinflussen und schränken daher die Verwendungsmöglichkeiten der Pentafluorphenylsalze drastisch ein. Hinzu kommt, dass die Anionen, die am Stickstoff neben der C=O-Gruppe eine Pentafluorphenyl-Gruppe tragen, eine verhältnismäßig geringe Acidität bzw. eine verhältnismäßig hohe Basizität aufweisen. Aufgrund dieser Eigenschaft verfügen die Anionen über Donor-Eigenschaften, die bei einer Verwendung der Salze als Elektrolyt unvorteilhaft sind.

### Aufgabe

Aufgabe der vorliegenden Erfindung ist es, Salze umfassend neue, wenig basische fluorierte Imidanionen, die als Anionen in ionischen Flüssigkeiten verwendet werden können, sowie Verfahren zu ihrer Herstellung bereitzustellen, wobei unter wenig basisch solche Anionen verstanden werden, bei denen der pK_{A}-Wert der korrespondierenden Säure, gemessen in DMSO, kleiner oder gleich 15 ist.

Eine weitere Aufgabe der vorliegenden Erfindung ist es, ionische Flüssigkeiten umfassend die neuen, wenig basischen fluorierten Imidionen als Anionen und Imidazolium-, Imidazolidinium-, Benzimidazoliumionen, Alkyl-alkyliden-phosphoranen oder Aryl-alkyliden-phosphoranen als Kationen sowie Verfahren zu deren Herstellung bereitszustellen.

### Lösung der Aufgabe

Die Aufgabe des Bereitstellens von Salzen umfassend wenig basische fluorierte Imidanionen wird gelöst durch Verbindungen enthaltend
a) ein Anion der allgemeinen Formel 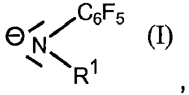 worin
   R¹ = -SO₂-R² darstellt, wobei
   R₂ eine verzweigte oder unverzweigte Alkylgruppe mit 1 bis 20 C-Atomen oder eine Aryl- oder Benzylgruppe darstellt und diese Alkyl-, Benzyl- oder Arylgruppe partiell oder vollständig fluoriert ist;
   und
b) ein Kation, ausgewählt aus
   anorganischen Kationen aus der Gruppe der Alkalikationen und Erdalkalikationen oder
   quaternären organischen Kationen.

Überraschend wurde gefunden, dass Salze umfassend die fluorierten Imidanionen I, welche eine Pentafluorphenylgruppe enthalten, im Gegensatz zu den bisher bekannten Imidanionen wenig basisch und in Form freier Ionen stabil sind. Unter freien Ionen werden hierbei Ionen verstanden, die im kristallinen oder geschmolzenen Zustand vollständig in ein Anion-Kation-Paar dissoziiert vorliegen. Als wenig basisch werden solche Imidanionen betrachtet, bei denen der pK_{A}-Wert der korrespondierenden Säure kleiner oder gleich 15 ist. Damit eignen sich die erfindungsgemäßen Imidanionen zur Herstellung ionischer Flüssigkeiten.

Erfindungsgemäße Anionen ⁻N(C₆F₅)R¹, bei denen R¹ und R² wie oben definiert sind, sind bevorzugt mehr als einfach fluoriert. Besonders bevorzugt sind solche Anionen ⁻N(C₆F₅)R¹, bei denen R¹ und R² wie oben definiert sind und bei denen Alkyl-, Aryl- bzw. Benzylgruppen perfluoriert sind.

Optional können die oben für R² angegebenen Reste, falls es sich dabei um Alkyl-, Aryl oder Benzylgruppen handelt, ihrerseits ein bis zwei Substituenten, ausgewählt aus Alkyl- und Arylgruppen, tragen.
Steht R² für eine Alkyl-, Aryl- oder Benzylgruppe und sind an diese Alkyl-, Aryl- oder Benzylgruppen weitere Alkylgruppen gebunden, so können diese weiteren Alkylgruppen linear oder verzweigt sein und enthalten 1 bis 20 C-Atome.
Steht R² für eine Alkyl-, Aryl- oder Benzylgruppe und sind an diese Alkyl-, Aryl- oder Benzylgruppe weitere Alkyl- oder Arylgruppen gebunden, so können diese weiteren Alkyl- oder Arylgruppen des Weiteren teilweise oder vollständig fluoriert sein.
Steht R² für eine Alkyl-, Aryl- oder Benzylgruppe und sind an diese Alkyl-, Aryl- oder Benzylgruppe zwei weitere Alkylgruppen oder zwei weitere Arylgruppen gebunden, so können diese beiden weiteren Alkylgruppen oder die beiden weiteren Arylgruppen identisch oder verschieden sein.

Ionische Flüssigkeiten, die -N(C₆F₅)R¹-Anionen enthalten, wobei R¹ wie oben definiert ist, weisen eine niedrigere Viskosität und niedrigere Schmelzpunkte auf als der Stand der Technik. Des Weiteren sind sie weniger flüchtig und besitzen eine hohe intrinsische Lipophilie und Löslichkeit für organische Substrate bzw. eine hohe Hydrophobie, d.h. die Nicht-Mischbarkeit mit bzw. geringe Sättigungskonzentration gegenüber Wasser.
Ursache für diese vorteilhaften Eigenschaften ist die im Vergleich zu Perfluoralkyl- und Perfluoralkylsulfonyl-Gruppen leichter polarisierbare Pentafluorphenylgruppe.

Organische Kationen werden bevorzugt ausgewählt aus der Gruppe quaternären Ammoniumionen, Phosphoniumionen, Guanidiniumionen, Imidazoliumionen, Imidazolidiniumionen, Benzimidazoliumionen und N-Organo-Pyridiniumionen.

Ammoniumionen werden besonders bevorzugt ausgewählt aus Verbindungen der Formel

[NR³R⁴R⁵R⁶]⁺ **(II),**

worin
R³, R⁴ und R⁵ unabhängig voneinander eine lineare oder verzweigte Alkylgruppe mit 1 bis 20 C-Atomen oder eine Arylgruppe oder eine Benzylgruppe darstellen und
R⁶ eine lineare oder verzweigte Alkylgruppe mit 1 bis 20 C-Atomen darstellt.

Phosphoniumionen werden besonders bevorzugt ausgewählt aus Verbindungen der Formel

[PR³R⁴R⁵R⁷]⁺ **(III**),

worin R³, R⁴ und R⁵ die oben aufgeführten Bedeutungen haben und
R⁷ eine lineare oder verzweigte Alkylgruppe mit 1 bis 20 C-Atomen oder eine Arylgruppe oder eine Benzylgruppe darstellt.

Guanidiniumionen werden besonders bevorzugt ausgewählt aus Verbindungen der Formel 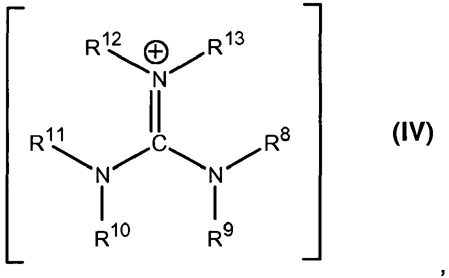 worin
R⁸, R⁹, R¹⁰, R¹¹, R¹² und R¹³ unabhängig voneinander ein H-Atom oder eine lineare oder verzweigte Alkylgruppe mit 1 bis 20 C-Atomen oder eine Arylgruppe darstellen.

Besonders bevorzugt sind im Rahmen der vorliegenden Erfindung solche Guanidiniumionen, bei denen R⁸, R⁹, R¹⁰, R¹¹, R¹² und R¹³ unabhängig voneinander eine lineare oder verzweigte Alkylgruppe mit 1 bis 20 C-Atomen darstellen.

Imidazoliumionen werden bevorzugt ausgewählt aus Ionen der allgemeinen Formel 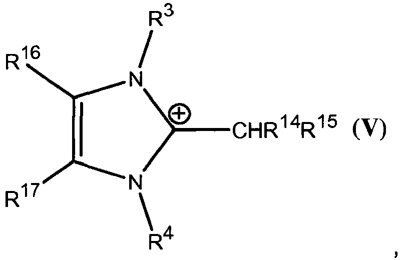 worin
R³ und R⁴ wie oben definiert sind und
R¹⁴, R¹⁵, R¹⁶ und R¹⁷ unabhängig voneinander jeweils für ein H-Atom, eine verzweigte oder unverzweigte Alkylgruppe mit 1 bis 20 C-Atomen, eine Aryl- oder eine Benzylgruppe stehen.

Imidazolidiniumionen werden bevorzugt ausgewählt aus Ionen der allgemeinen Formel 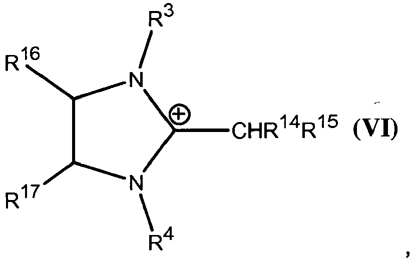 worin R³, R⁴, R¹⁴, R¹⁵, R¹⁶ und R¹⁷ wie oben definiert sind.

Benzimidazoliumkationen werden bevorzugt ausgewählt aus Ionen der allgemeinen Formel 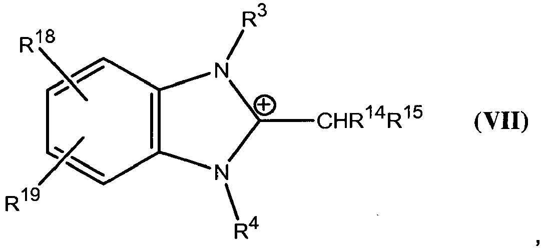 worin R³, R⁴, R¹⁴ und R¹⁵ wie oben definiert sind und
R¹⁸ und R¹⁹ unabhängig voneinander für ein H-Atom, F, Cl, eine lineare oder verzweigte Alkylgruppe mit 1 bis 20 C-Atomen, eine Aryl- oder eine Benzylgruppe stehen.

N-Organo-Pyridiniumionen werden bevorzugt ausgewählt aus Kationen der allgemeinen Formel 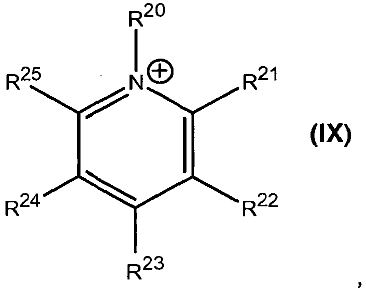 worin
R²⁰ eine lineare oder verzweigte Alkylgruppe mit 1 bis 20 C-Atomen, eine Aryl- oder eine Benzylgruppe darstellt, und
R²¹, R²², R²³, R²⁴ und R²⁵ unabhängig voneinander ein H-Atom, F, Cl oder eine lineare oder verzweigte Alkylgruppe mit 1 bis 20 C-Atomen darstellen.

Besonders bevorzugt sind im Rahmen der vorliegenden Erfindung solche N-Organo-Pyridiniumionen, bei denen R²⁰ eine verzweigte oder unverzweigte Alkylgruppe mit 1 bis 20 C-Atomen darstellt.

Bevorzugt werden solche erfindungsgemäßen Salze in ionischen Flüssigkeiten verwendet, bei denen R¹ des -N(C₆F₅)R¹-Anions gemäß obiger Definition eine fluorierte Arylsulfonyl- oder Alkylsulfonylgruppe ist.
Wird als Kation einer erfindungsgemäßen ionischen Flüssigkeit ein Imidazolium-, Imidazolidinium- oder Benzimidazoliumion gewählt, so sind solche Kationen bevorzugt, bei denen die Reste R³ und R⁴ gemäß den oben angegebenen Definitionen unterschiedlich sind.
Wird als Kation dagegen ein Ammonium- oder Phosphoniumion gewählt, so sind unsymmetrisch substituierte Ionen gemäß den oben aufgeführten Formeln (II) bzw. (III) bevorzugt. Unter unsymmetrisch substituiert werden dabei solche Ammonium- bzw. Phosphoniumionen verstanden, bei denen die Reste R³, R⁴, R⁵, R⁶, bzw. R⁷ gemäß den obigen Definitionen so gewählt sind, das Kation keine Spiegelachse senkrecht zur Molekülebene aufweist.

Die Aufgabe, ein Verfahren zur Herstellung nicht basischer perfluorierter Bisorganylamide bereitzustellen, wird erfindungsgemäß gelöst durch die Reaktion des Pentafluorphenylanilid(2-)-Synthons [N-C₆F₅]2⁻ mit einem Sulfonsäurederivat in einem organischen Lösungsmittel.

Dies geschieht erfindungsgemäß durch Umsetzung von H₂N-C₆F₅ mit einem Metallierungsreagenz (Metallamid, Metallhydrid, Metallalkyl, Metallhydroxid, Metallcarbonat, Metall (elementar)/Metall vorzugsweise Alkali- oder Erdalkalimetall). Anschließende Reaktion des metallierten Anilids M-NH-C₆F₅, wobei "M" für Metall steht, mit einem Elektrophil
- aus der Gruppe der R²-substituierten Sulfonsäurechloride, -fluoride, -ester, - anhydride, wobei R² wie oben beschrieben definiert ist
   in Gegenwart einer Hilfsbase B, die mindestens so basisch ist wie das Anion X, 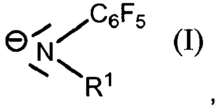 worin R¹ wie oben definiert ist.

Grund: Die Reaktion erfolgt nach folgender Stöchiometrie mit höchster Ausbeute:

M-NH-C₆F₅ + R¹X + B → M-N-(C₆F₅)R¹ + [HB]X

bzw.

M-NH-C₆F₅ + R²-SO₂-X + B → M-N-(C₆F₅)-SO₂-R² + [HB]X

B kann sein: eine Neutralbase wie beispielsweise, aber nicht erschöpfend, ein Alkylamin NH₃₋ₓR¹x, Guanidin oder eine salzartige Base aus der Klasse der Metallierungsmittel (Metallamid, Metallhydrid, Metallalkyl, Metallhydroxid, Metallcarbonat, Metall (elementar)/Metall vorzugsweise Alkali- oder Erdalkalimetall), besoders bevorzugt sind starke und nicht besonders nukleophile Basen, z.B. Na[N(SiMe₃)₂] oder K[O^{t}Bu].

Beispielhaft sei hierfür die Umsetzung des Trifluormethylsulfonsäureanhydrids mit Pentafluorphenylanilin in Gegenwart von 2 Äquivalenten Na-*bis*-trimethylsilylamid Na[N(SiMe₃)₂] genannt:
Hierbei wird ein Äquivalent Pentafluorphenylanilin in THF gelöst, bei -80 °C mit zwei Äquivalenten Na[N(SiMe₃)₂] umgesetzt und das Reaktionsgemisch mit einem Äquivalent des Elektrophils (Trifluormethylsulfonsäureanhydrid) umgesetzt. Die Reaktion liefert das Lithiumsalz des Anions I mit R¹ = SO₂CF₃, die Aufarbeitung mit wässriger HCl bei einem pH-Wert von 2 bis 5 liefert die korrespondierende NH-Säure dieses Anions.

Für den Fachmann ist ohne Weiteres ersichtlich, dass sich andere erfindungsgemäße Anionen (I) in analoger Weise herstellen lassen. Er kann diese anderen erfindungsgemäßen Anionen mit Hilfe seines Fachwissens und ohne den Schutzbereich der Patentansprüche zu verlassen anwenden. Die korrespondierenden Säuren (X) lassen sich durch Protonierung bei einem pH-Wert von 0 bis 6 gewinnen.

Alle in der vorliegenden Offenbarung aufgeführten Reste R² bis R²⁵ können - wie ausgeführt - u.a. für eine lineare oder verzweigte Alkylgruppe mit 1 bis 20 C-Atomen stehen. Es ist hervorzuheben, dass in allen diesen Fällen solche Alkylgruppen besonders bevorzugt sind, die 1 bis 4 C-Atome enthalten, d.h. Methyl, Ethyl, n-Propyl, iso-Propyl, n-Butyl, 2-Butyl und tert.-Butyl.

Erfindungsgemäße ionische Flüssigkeiten werden hergestellt durch Umsetzung der entsprechenden korrespondierenden Säure 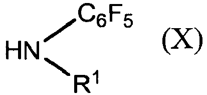 des Anions I 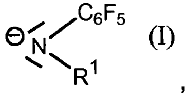 worin
R¹ = -SO₂-R² darstellt, und wobei
R₂ eine verzweigte oder unverzweigte Alkylgruppe mit 1 bis 20 C-Atomen oder eine Aryl- oder Benzylgruppe darstellt und diese Alkyl-, Benzyl- oder Arylgruppe partiell oder vollständig fluoriert ist;
mit einer korrespondierenden Verbindung des gewünschten Kations, und zwar
- quaternären Ammoniumhydroxiden, falls als Kation ein quaternäres Ammoniumkation gemäß obiger Definition gewünscht ist,
- Guanidiniumionen gemäß obiger Definition (über Ionenaustausch), falls als Kation ein quaternäres Guanidiniumion gewünscht ist,
- Alkyl-alkyliden-phosphoranen oder Aryl-alkyliden-phosphoranen (P-Yliden), falls als Kation ein Phosphoniumkation gemäß obiger Definition gewünscht ist, oder
- einem Keten-N,N-diacetal, falls als Kation ein 2-Alkyl- substituiertes Imidazolium-, Imidazolidinium- oder Benzimidazolium-Kation gemäß einer der obigen Definitionen gewünscht ist,
- einem Salz eines N-Organo-Pyridiniumions gemäß obiger Definition, falls als Kation ein N-Organo-pyridiniumkation gewünscht ist,
in einem organischen Lösungsmittel.

Das Lösungsmittel für die Umsetzung wird bevorzugt ausgewählt aus der Gruppe der reinen aliphatischen, der ungesättigten und der aromatischen Kohlenwasserstoffe, beispielsweise Toluol, partiell oder voll halogenierte Kohlenwasserstoffe (z.B. Chlorbenzol, Chloroform, Tetrachlorkohlenstoff, FCKW, FKW, Frigene), der organischen Amine, Ether, Alkohole (optional im Gemisch mit Wasser), Ketone, DMF, DMSO, HMPT, der organischen Carbonate, Carbonsäureamide und Carbonsäureester und Tetraalkylharnstoffe.
Der Stand der Technik kennt ionische Flüssigkeiten, die quaternäre 2-Alkylsubstituierte Imidazolium-, Imidazolidinium- und Benzimidazoliumkationen enthalten. Nachteilig bei den bekannten Verfahren zur Herstellung dieser ionischen Flüssigkeiten ist jedoch, dass sich die ionischen Flüssigkeiten mit ihrer Hilfe nicht direkt, quantitativ und wasserfrei herstellen lassen.

Keten-N,N-diacetale sind dem Fachmann bekannt; er weiß auch, dass sie sich zu 2-Alkyl-substituierten Imidazolium-, Imidazolidinium- und Benzimidazoliumsalzen reprotonieren lassen. Es ist weiterhin Stand der Technik, dass nucleophile Carbene sich zu den entsprechenden 2-H-substituierten Imidazolium-, Imidazolidinium- und Benzimidazoliumsalzen protonieren lassen.
Neu und überraschend ist jedoch, dass die Umsetzung von Keten-N,N-diacetalen mit den korrespondierenden Säure H[N(C₆F₅)R¹] der erfindungsgemäßen Anionen [N(C₆F₅)R¹]⁻ direkt und quantitativ in einem organischen Lösungsmittel zu den entsprechenden wasserfreien ionischen Flüssigkeiten führt. Dies ist nachfolgend beispielhaft und schematisch für ein 1,3-Dialkyl-2-methylenimidazol als Vertreter eines Keten-N,N-diacetals gezeigt:

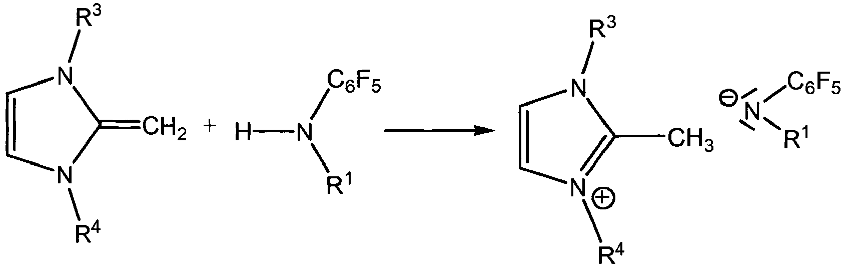

Hierbei sind R¹, R³ und R⁴ wie oben definiert. Es ist für den Fachmann ohne Weiteres ersichtlich, dass diese Reaktion in analoger Weise auch mit den entsprechenden 1,3-Dialkyl-2-alkylen-imidazolinen, 1,3-Diaryl-2-alkylen-imidazolinen, 1,3-Dialkyl-2-alkylen-benzimidazolen, 1,3-Dialkyl-2-benzilyden-imidazolen und weiteren Vertretern der Klasse der Keten-N,N-diacetale durchführbar ist.
Ebenso können die korrespondierenden Säuren (X) der erfindungsgemäßen Anionen (I) mit Alkyl-alkyliden-phosphoranen oder Aryl-alkyliden-phosphoranen direkt und quantitativ in einem organischen Lösungsmittel zu den entsprechenden ionischen Flüssigkeiten umgesetzt werden. Dieses Verfahren ist beispielhaft in Ausführungsbeispiel 15 für die Herstellung von Methyltriphenylphosphoniumdecafluordiphenylamid gezeigt und kann auch für die Herstellung anderer Organophosphoniumimidsalze verwendet werden.

Des Weiteren ist für den Fachmann ohne Weiteres ersichtlich, dass sich die Keten-N,N-diacetale auch mit anderen O-H-, C-H- und N-H-, Halogenwasserstoffsäuren sowie Fluoroborsäuren, Fluorokieselsäuren, Fluorosphorsäuren, Fluoroarsensäuren, Fluoroantimonsäuren, Fluoroschwefelsäuren, weiterhin alle Mineralsäuren und Oxosäuren der Nichtmetalle und Metalle mit einem pK_{A} Wert kleiner gleich 15 gemessen in DMSO in einem organischen Lösungsmittel zu ionischen Flüssigkeiten umsetzen lassen.
Beispielhaft, aber nicht erschöpfend, seien als Säuren HC(SO₂CF₃)₃, HN(SO₂CF₃)₂, Pentafluorphenol, HBF₄, H₂SiF₆, HPF₆, HAsF₆, HSbF₆, HSO₃F, HF, HCl, HBr, HI sowie Oxosäuren, ausgewählt aus Stickstoffsäuren, Schwefelsäuren, Chlorsäuren, Bromsäuren, Phosphorsäuren, Chromsäuren, Titansäuren und Wolframsäuren, Vanadinsäuren, Molybdänsäuren genannt.

Der Fachmann kann die oben beschriebenen Klassen der Protonensäuren mit Hilfe seines allgemeinen Fachwissens mit Keten-N,N-diacetalen zu 2-Alkyl-imidazolium-basierten und mit Alkyl-alkyliden-phosphoranen bzw. Aryl-alkyliden-Phosphoranen zu Organophosphononium-basierten ionischen Flüssigkeiten umsetzen.

2-Alkyl- substituierte quaternäre Imidazolium-, Imidazolidinium- und Benzimidazoliumsalze sind besonders vorteilhafte Kationen in ionischen Flüssigkeiten, da sie weniger CH-acide und deshalb stabiler gegenüber einem nucleophilen Angriff sind als die entsprechenden 2-H-Imidazoliumsalze, die bekanntermaßen über Carbene erhältlich sind.

Das Lösungsmittel für die Umsetzung wird ausgewählt aus der Gruppe der reinen aliphatischen, der ungesättigten und der aromatischen Kohlenwasserstoffe, beispielsweise Toluol, partiell oder voll halogenierte Kohlenwasserstoffe (z.B. Chlorbenzol, Chloroform, Tetrachlorkohlenstoff, FCKW, FKW, Frigene), der organischen Amine, Ether, Alkohole (optional im Gemisch mit Wasser), Ketone, DMF, DMSO, HMPT, der organischen Carbonate, Carbonsäureamide und Carbonsäureester und Tetraalkylharnstoffe.

Die erfindungsgemäßen ionischen Flüssigkeiten können als Ersatz für organische Lösungsmittel in großtechnischen Synthesen verwendet werden. Durch ihren geringen Dampfdruck kommt es zu einer Verminderung gasförmiger Emissionen. Prozesse, bei denen ionische Flüssigkeiten als Lösungsmittel verwendet werden, zeichnen sich durch ein niedrigeres Expositionspotenzial für das beteiligte Personal sowie eine geringere Explosionsgefahr aus. Dank ihrer hervorragenden Lösungseigenschaften werden ionische Flüssigkeiten als Lösungsmittel für Substanzen, die mit konventionellen organischen Lösungsmitteln schwer in Lösung zu bringen sind, wie z.B. Cellulose, eingesetzt.
Bedingt durch ihre Lösungs- und Absorptionseigenschaften sind ionische Flüssigkeiten ferner als Extraktionsmittel geeignet. Sie können dazu verwendet werden, um azeotrope Gemische aufzutrennen. Des Weiteren sind sie vorteilhaft als mobile und/oder stationäre Phasen in der Chromatographie zu verwenden, beispielsweise für GC, LC, HPLC, Ionenchromatographie.

Weiterhin werden ionische Flüssigkeiten mit elektrochemisch inerten fluorierten Anionen als Elektrolytsysteme für Batterien, galvanische Elemente, Brennstoffzellen und Akkumulatoren (beispielsweise Lithiumionenakkus und Lithiumionen-Elektrolyte) verwendet.
Auch als Wärmeträger in solartechnischen Anlagen lassen sie sich verwenden. Manche-quaternäre Imidazoliumsalze finden Anwendung als Mikrobiozide. Einige quaternäre Ammoniumsalze fungieren als kationische Tenside.

### Ausführungsbeispiele

### 1. 1-n-Butyl-2,3-dimethyl-imidazoliumchlorid

Das Produkt ist literaturbekannt, vgl. V. Farmer, T. Welton, Green Chemistry 2002, 4,97-102.

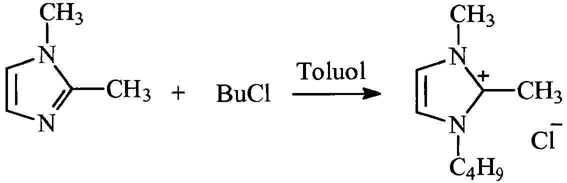

Zu einer Lösung von 59.43 g (0.618 mol) 1,2-Dimethylimidazol in 50 ml Toluol werden 71.05 ml (0.680 mol) n-Butylchlorid gegeben. Die Reaktionsmischung wird 24 h unter Rückfluss erhitzt, wobei sich ein Zweiphasensystem bildet. Die Mischung wird für 8 h bei -30°C gelagert, wobei sich ein weißer Feststoff bildet. Der erhaltene weiße Feststoff wird aus warmem Acetonitril umkristallisiert. Ausbeute: 70.00 g (60%) (weißer, hygroskopischer Feststoff).

**¹H-NMR** (CDCl₃, 200 MHz): δ = 1.30 (s, 3 H, NCH₂CH₂CH₂C*H*₃), 1.74 (sext, 2 H, NCH₂CH₂C*H*₂CH₃), 2.16 (quint, 2 H, NCH₂C*H*₂CH₂CH₃), 3.20 (s, 3 H, NC*H*₃), 4.42 (s, 3 H, CC*H*₃), 4.62 (t, 2 H, NC*H*₂CH₂CH₂CH₃), 8.01 (d, 1 H, BuNC*H*), 8.25 (d, 1 H, (MeNC*H*).

### 2. 1-n-Butyl-3-methyl-2-methylenimidazolin

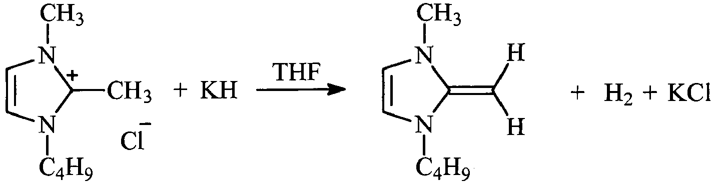

In einen 250 ml Kolben werden in der Glovebox 9.43 g (49.60 mmol) 1-n-Butyl-2,3-dimethyl-imidazoliumchlorid und 4.97 g (123.91 mmol) KH vermischt. Die Lösung wird mit 100 ml THF versetzt und für 60 h bei Raumtemperatur gerührt. Die erhaltene Reaktionsmischung wird über Celite filtriert und von der Lösung alle bei Raumtemperatur flüchtigen Bestandteile abkondensiert. Anschließend wird bei 160°C / 0.1 mbar das Produkt in ein mit Stickstoff gekühltes Schlenkrohr destilliert. Ausbeute: ca. 70% (farblose, extrem feuchtigkeitsempfindliche Flüssigkeit, die sich bei Raumtemperatur unter partieller Zersetzung rasch gelb färbt).

**¹H-NMR** (C₆D₆, 300 MHz): δ = 0.92 (t, 3 H, NCH₂CH₂CH₂CH₃), 1.30 (sext, 2 H, NCH₂CH₂CH₂CH₃), 1.60 (quint, 2 H, NCH₂CH₂CH₂CH₃), 2.75 (s, 3 H, NCH₃), 2.77 (s, 1 H, CCH₂), 2.83 (s, 1 H, CCH₂), 3.22 (t, 2 H, NCH₂CH₂CH₂CH₃), 5.75 (s, 1 H, BuNCH), 5.80 (s, 1 H, MeNCH).
**¹³C**-**NMR** (C₆D₆, 50 MHz): δ = 13.88 (NCH₂CH₂CH₂*C*H₃), 20.21 (NCH₂CH₂*C*H₂CH₃), 29.65 (NCH₂*C*H₂CH₂CH₃), 32.52 (NCH₃), 39.84 (C*C*H₂), 45.80 (N*C*H₂CH₂CH₂CH₃), 112.07 (BuN*C*H), 113.06 (MeN*C*H), 151.98 (N*C*N)

### 3. 1-Ethyl-3-methyl-2-methylenimidazolin

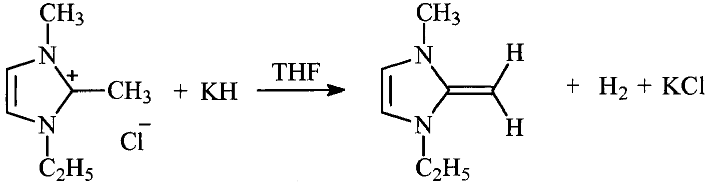

In einen 250 ml Kolben werden in der Glovebox 5.96 g (37.12 mmol) 1-Ethyl-2,3-dimethyl-imidazoliumchlorid und 2.98 g (74.24 mmol) KH vermischt. Die Lösung wird mit 100 ml THF versetzt und für 60 h bei Raumtemperatur gerührt. Die erhaltene Reaktionsmischung wird über Celite filtriert und von der Lösung alle bei Raumtemperatur flüchtigen Bestandteile abkondensiert. Anschließend wird bei 160°C / 0.1 mbar das Produkt in ein mit Stickstoff gekühltes Schlenkrohr destilliert. Ausbeute: ca. 70% (farblose, extrem feuchtigkeitsempfindliche Flüssigkeit, die sich bei Raumtemperatur rasch gelb färbt).

**¹H-NMR** (C₆D₆, 200 MHz): δ = 0.92 (t, 3 H, NCH₂C*H*₃), 2.56 (s, 3 H, NC*H*₃), 2.65 (s, 2 H, CC*H*₂), 3.00 (q, 2 H, NC*H*₂CH₃), 5.56 (d, 1 H, EtNC*H*), 5.61 (d, 1 H, MeNC*H*).
**¹³C**-**NMR** (C₆D₆, 50 MHz): δ = 12.64 (NCH₂*C*H₃), 32.49 (C*C*H₂), 39.82 (N*C*H₃), 40.42 (N*C*H₂CH₃), 110.94 (EtN*C*H), 113.27 (MeN*C*H), 151.80 (N*C*N).

### 4. 1,3-Di(iso-propyl)imidazolium-pentafluorphenolat (nicht erfindungsgemäß)

Das N-heterocyclische Carben sowie das Verfahren zur Herstellung 2-H-Imidazolbasierter Ionischen Flüssigkeiten sind aus der WO 01/77081 A1 bekannt.

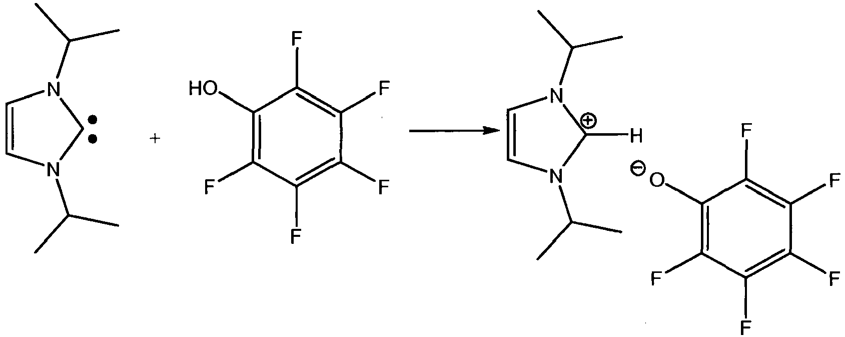

Zu einer auf -78°C gekühlten Lösung von 1.50 g (8.15 mmol) Pentafluorphenol in Diethylether gibt man mittels einer Spritze 1.25 ml (8.15 mmol) 1,3-Di(*iso-*propyl)imidazol-2-yliden. Die Reaktionsmischung wird über einen Zeitraum von 5h auf Raumtemperatur erwärmt und 8h bei Raumtemperatur gerührt. Anschließend werden alle flüchtigen Bestandteile im Vakuum entfernt, der Rückstand mit 20 ml Hexan gewaschen und anschließend im Vakuum getrocknet. Man erhält das gewünschte Produkt als weißen Feststoff, der bei -30°C aus Dichlormethan umkristallisiert werden kann.

**¹H-NMR** (D₃CCN, 300 MHz): δ = 1.50 (d, 12 H, NCH(C*H*₃)₂), 4.60 (sept, 2 H, NC*H*), 7.50 (s, 2 H, NC*H*C*H*N), 9.40 (s, 1 H, NC*H*N).
**¹³C-NMR** (D₃CCN, 50 MHz): 22.79 ppm (NCH(*C*H₃)₂), 53.98 ppm (N*C*H(CH₃)₂), 121.31 ppm (N*C*H*C*HN), 135.11 ppm (N*C*N).
**¹⁹F-NMR** (D₃CCN, 282 MHz): δ = -197.11 (t, 1 F, *p*-F), -174.06 (2 F, *m*-F), -173.99 (2 F, *o*-F).

### 5. 1,3-Di(iso-propyl)imidazolium-decafluordiphenylimid (nicht erfindungsgemäß)

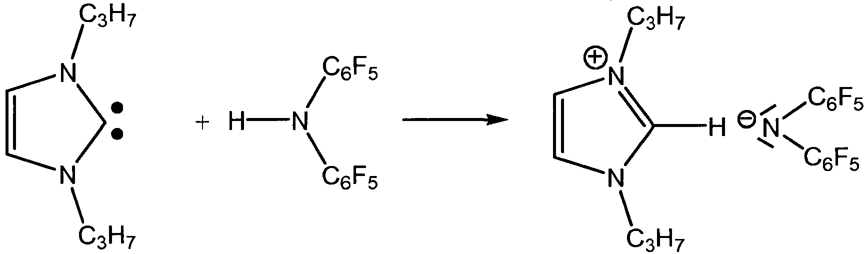

In einem Schlenkkolben werden bei -78 °C zu einer Lösung von 0.524 g (2.86 mmol) Decafluordiphenylamin 5 ml Et₂O unter Rühren 0.2 ml (1.31 mmol) 1,3-Di(iso-propyl)imidazol-2-yliden gegeben. Die Mischung wird innerhalb von 19 h auf Raumtemperatur gebracht und das Lösungsmittel im Vakuum entfernt. Der Rückstand wird mit 10 ml *n*-Hexan digeriert, filtriert, mit 10 ml *n*-Pentan gewaschen und im Vakuum getrocknet. Man erhält 0.530 mg (1.06 mmol, 80.9%) Di(*iso-*propyl)imidazolium-decafluordiphenylimid in Form eines leicht gelblichen Feststoffs. Durch Umkristallisation aus 10 ml Et₂O bei -30 °C erhält man farblose Kristalle, die zur Röntgenstrukturbestimmung geeignet sind.
**Fp.:** 109 °C
Massenspektrum (EI):
m/z = 43 (27.72, *i*-Pr+), 110 (12.51, Im^{*i*Pr}-H - *i*-Pr), 152 (11.13, Im^{*i*Pr}H+), 349 (28.42, (C₆F₅)₂NH⁺)
**C, H, N-Analyse:** gefunden (berechnet)
C: 50.14%(50.31%), H: 3.43%(3.42%), N. 8.72%(8.38%)
**IR:** cm⁻¹: 30.46s, 2723.66s, 2362.95s, 1618.38s, 1305.89s, 1263.45s, 1018.48m, 968.33m,
939.39s, 814.01s, 740.71s, 557.46m
**¹H-NMR** (D₃CCN, 300 MHz): δ = 0.83 ppm (d, 12 H, NCH(C*H*₃)₂), 3.90 ppm (sept, 2 H, NC*H*)" 5.85 ppm (s, 2 H, NC*H*C*H*N), 9.40 (s, 1 H, NC*H*N).
**¹³C**-**NMR** (D₃CCN, 50 MHz): 21.96 ppm (NCH(*C*H₃)₂), 52.79 ppm (N*C*H(CH₃)₂), 118.40 ppm (NCH*C*HN), 137.98 ppm (NCN).
**¹⁹F-NMR** (D₃CCN, 282 MHz): δ = -183.35 ppm (t, 1 F, *p*-F), -169.80 ppm (2 F, *m-*F), -160.94 ppm (d, 1F, *o*-F).

### Röntgenstrukturanalyse:

Mo-Kα-Strahlung (Wellenlänge: 71.069 nm), 20 °C
*Summenformel:* C₂₁H₁₇F₁₀N₃
*Molmasse:* 501.38 g/mol
Zahl der Formeleinheiten: 4
*Elementarzelle:* a = 14.1670(16) Ǻ, b = 11.0290(10) Ǻ, c = 13.6160(14) Ǻ α = 90.00°, β = 91.0680(10)°
Kristallsystem: monoklin
*Raumgruppe*: C2/c
Röntgenographische Dichte: 1.566 g/ml
Absorptionskoeffizient: 0.155 mm₋₁
*Fp.:* 109 °C
Gemessene Reflexe: 10551
Beobachtete Reflexe: 1626
Zahl der unabhängigen Reflexe:2269
*Messbereich (θ):* 2.34 - 26.76°
Parameterzahl: 190
*R₁; R₂ :* 0.0351; 0.0934
Restelektronendichte (min., max.): -0.235, 0.211

**¹³C-NMR** (D₃CCN, 50 MHz): δ = 10.07 (NCH₂CH₂CH₂*C*H₃), 13.78 (NCH₂CH₂*C*H₂CH₃), 20.12 (C*C*H₃), 32.33 (N*C*H₃), 35.72 (NCH₂*C*H₂CH₂CH₃), 48.97 (N*C*H₂CH₂CH₂CH₃), 121.92 (BuN*C*H), 123.38 (MeN*C*H), 121.70 (q, *C*OO), 145.42 (N*C*N), 160.27 (q, *C*F₃).
**¹⁹F-NMR** (D₃CCN, 282 MHz): δ = -75.58 (s, 3 F, OOCC*F*₃).

### 6. 1-n-Butyl-2,3-dimethylimidazolium-trifluoracetat (nicht erfindungsgemäß)

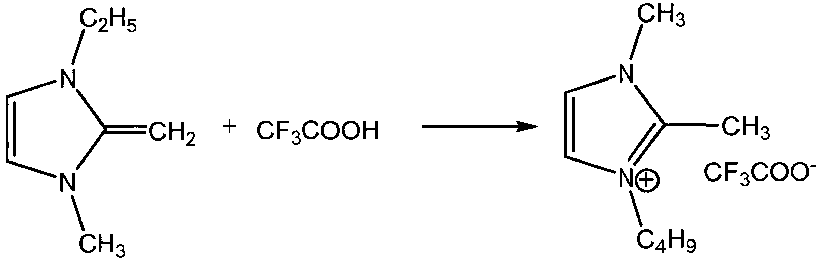

Zu einer auf -78°C gekühlten Lösung von 1.38 g (12.09 mmol) Trifluoressigsäure in Diethylether gibt man mittels einer Spritze 1.84 ml (12.09 mmol) 1-n-Butyl-3-methyl-2-methylenimidazolin. Die Reaktionsmischung wird langsam auf Raumtemperatur erwärmt, wobei sich ein weißer Feststoff bildet und über Nacht bei Raumtemperatur gerührt. Anschließend werden alle flüchtigen Bestandteile im Vakuum entfernt, der Rückstand mit 20 ml Hexan gewaschen und anschließend im Vakuum getrocknet. Man erhält das gewünschte Produkt als weißen Feststoff, der aus Dichlormethan umkristallisiert werden kann.

**¹H-NMR** (D₃CCN, 300 MHz): δ = 0.92 (t, 3 H, NCH₂CH₂CH₂*C*H₃), 1.33 (sext, 2 H, NCH₂CH₂*CH*₂CH₃), 1.72 (quint, 2 H, NCH₂C*H*₂CH₂CH₃), 2.51 (s, 3 H, NC*H*₃), 3.71 (s, 3 H, CC*H*₃), 4.04 (t, 2 H, NC*H*₂CH₂CH₂CH₃), 7.23 (d, 1 H, BuNC*H*), 7.47 (d, 1 H, MeNC*H*).
**¹³C-NMR** (D₃CCN, 50 MHz): δ = 10.07 (NCH₂CH₂CH₂*C*H₃), 13.78 (NCH₂CH₂*C*H₂CH₃), 20.12 (C*C*H₃), 32.33 (N*C*H₃), 35.72 (NCH₂*C*H₂CH₂CH₃), 48.97 (N*C*H₂CH₂CH₂CH₃), 121.92 (BuN*C*H), 123.38 (MeN*C*H), 121.70 (q, *C*OO), 145.42 (N*C*N), 160.27 (q, *C*F₃).
**¹⁹F-NMR** (D₃CCN, 282 MHz): δ = -75.58 (s, 3 F, OOCC*F*₃).

### 7. 1-n-Butyl-2,3-dimethylimidazolium-decafluordiphenylimid (nicht erfindungsgemäß)

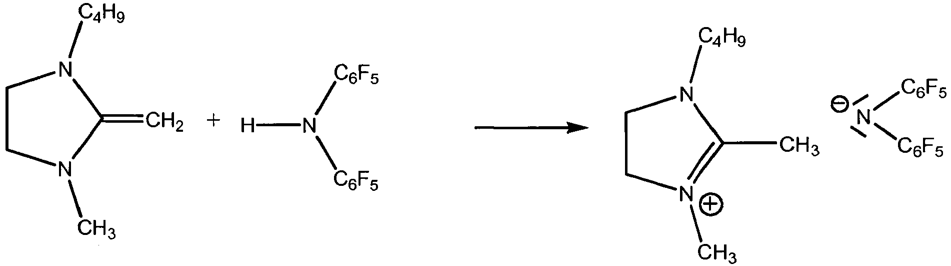

Zu einer auf -78°C gekühlten Lösung von 4.49 g (12.85 mmol) Decafluordiphenylamin in Diethylether gibt man mittels einer Spritze 1.96 ml (12.85 mmol) 1-n-Butyl-3-methyl-2-methylenimidazolin. Die Reaktionsmischung wird über einen Zeitraum von 5h auf Raumtemperatur erwärmt und 8h bei Raumtemperatur gerührt, wobei eine dunkelbraune Lösung entsteht. Anschließend werden alle flüchtigen Bestandteile im Vakuum entfernt, der Rückstand mit 20 ml Pentan gewaschen und anschließend im Vakuum getrocknet. Man erhält das gewünschte Produkt als braunes Öl.

**¹H-NMR** (C₆D₆, 300 MHz): δ = 0.73 (t, 3 H, NCH₂CH₂CH₂C*H*₃), 0.89 (sext, 2 H, NCH₂CH₂C*H*₂CH₃), 1.14 (quint, 2 H, NCH₂C*H*₂CH₂CH₃), 1.59 (s, 3 H, NC*H*₃), 2.86 (s, 3 H, CC*H*₃), 3.19 (t, 2 H, NC*H*₂CH₂CH₂CH₃), 6.63 (s, 1 H, BuNC*H*)*,* 6.67 (s, 1 H, MeNC*H*).
**¹³C**-**NMR** (C₆D₆, 50 MHz): δ = 7.87 (NCH₂CH₂CH₂*C*H₃), 13.10 (NCH₂CH₂*C*H₂CH₃), 19.46 (C*C*H₃), 31.32 (N*C*H₃), 33.91 (NCH₂*C*H₂CH₂CH₃), 47.97 (N*C*H₂CH₂CH₂CH₃), 120.66 (BuN*C*H), 122.27 (MeN*C*H), 142.84 (N*C*N), 130.83 - 140.5 (m, C(Ar^{F}))_{.}
**¹⁹F-NMR** (C₆D₆, 282 MHz): δ = -185.64 (m, 2 F, p-F), -170.45 (t, 4 F, o-F), -161.12 (dd, 4 F, m-F).

### Röntgenstrukturanalyse:

Mo-K_{α}-Strahlung (Wellenlänge: 71.069 nm), 20 °C
*Summenformel:* C₂₁H₁₇F₁₀N₃
*Molmasse:* 501.38 g/mol
*Zahl der Formeleinheiten: 2*
*Elementarzelle:* a = 9.8625(11) Ǻ, b = 10.3192(12) Ǻ, c = 10.8965(12) Ǻ α = 81.843(13)°, β = 80.651 (13)°, γ = 75.228(13)°
*Kristallsystem:* triklin
*Raumgruppe:* P1̅
*Röntgenographische Dichte:* 1.583 g/ml
*Absorptionskoeffizient:* 0.157 mm⁻¹
*Fp.:* 109 °C
*Gemessene Reflexe*: 10424
*Verwendete Reflexe*: 8000
*Zahl der unabhängigen Reflexe*:3839
*Messbereich (θ)*: 2.05 - 26.01 °
*Parameterzahl*: 375
*R₁; R₂:* 0.0337; 0.0914
*Restelektronendichte (min., max.)*: -0.158, 0.193

### 8. 1-Ethyl-2,3-dimethylimidazolium-pentafluorphenolat (nicht erfindungsgemäß)

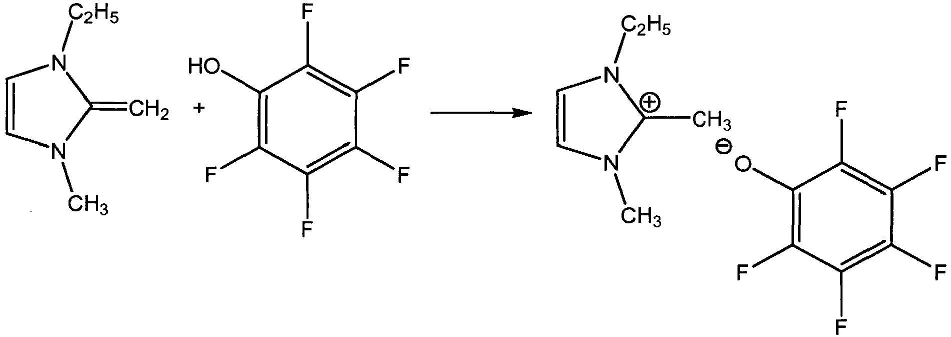

Zu einer auf -78°C gekühlten Lösung von 2.20 g (11.95 mmol) Pentafluorphenol in Diethylether gibt man mittels einer Spritze 1.48 ml (11.95 mmol) ) 1-Ethyl-3-methyl-2-methylenimidazolin. Die Reaktionsmischung wird über einen Zeitraum von 5h auf Raumtemperatur erwärmt und 8h bei Raumtemperatur gerührt. Der ausgefallene farblose Feststoff wird abfiltriert, mit zweimal 20 ml Diethylether gewaschen und anschließend im Vakuum getrocknet. Man erhält das gewünschte Produkt als farblosen Feststoff, der bei 144°C schmilzt.

**¹H-NMR** (D₃CCN, 300 MHz): δ = 1.3/ (t, 3 H, NCH₂C*H*₃), 2.50(s, 3 H, NC*H*₃), 3.70 (s, 3 H, CC*H*₃), 4.08 (q, 2 H, NC*H*₂CH₃), 7.33 (d, 1 H, EtNC*H*), 7.35 (d, 1 H, MeNC*H*).
**¹³C**-**NMR** (D₃CCN, 75 MHz): δ = 9.90 (NCH₂*C*H₃), 15.16 (C*C*H₃), 35.63 (N*C*H₃), 44.37 (N*C*H₂CH₃), 121.28 (EtN*C*H), 123.42 (MeN*C*H), 145.22 (N*C*N).
**¹⁹F-NMR** (D₃CCN, 282 MHz): δ = -197.97 (br, 1 F, p-F), 174.40-174.12 (m, 4 F, *o-*F + *m*-F).
**C, H, N-Analyse:** gefunden (berechnet)
C: 50.19%(50.65%), H: 4.59%(4.22%), N. 9.08%(9.09%)

### 9. 1-Ethyl-2,3-dimethylimidazolium-decafluordiphenylimid (nicht erfindungsgemäß)

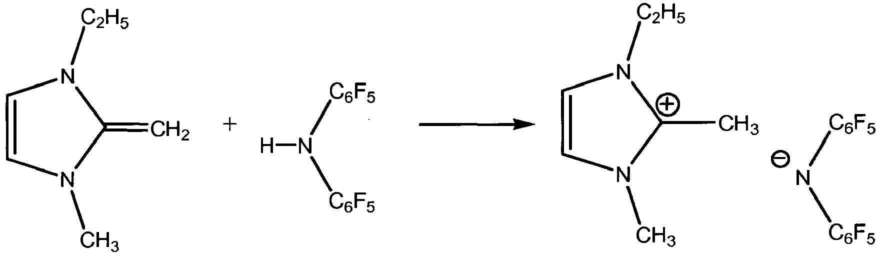

Zu einer auf -78°C gekühlten Lösung von 0.80 g (2.29 mmol) Decafluordiphenylamin in Diethylether gibt man mittels einer Spritze 0.28 ml (2.29 mmol) 1-Ethyl-3-methyl-2-methylenimidazolin. Die Reaktionsmischung wird über einen Zeitraum von 5h auf Raumtemperatur erwärmt und 8h bei Raumtemperatur gerührt. Der ausgefallene hellgelbe Feststoff wird abfiltriert, mit zweimal 20 ml Diethylether gewaschen und anschließend im Vakuum getrocknet. Man erhält das gewünschte Produkt als farblosen Feststoff, der bei 98°C schmilzt.
**¹H-NMR** (D₃CCN, 300 MHz): δ = 1.37 (t, 3 H, NCH₂C*H*₃), 2.47 (s, 3 H, NC*H*₃), 3.68 (s, 3 H, CC*H*₃), 4.06 (q, 2 H, NC*H*₂CH₃), 7.24 (d, 1 H, EtNC*H*), 7.27 (d, 1 H, MeNC*H*).
**¹³C-NMR** (D₃CCN, 75 MHz): δ = 9.92 (NCH₂*C*H₃), 15.14 (C*C*H₃), 35.68 (N*C*H₃), 44.42 (N*C*H₂CH₃), 121.22 (EtN*C*H), 123.35 (MeN*C*H), 145.26 (N*C*N).
**¹⁹F-NMR** (D₃CCN, 282 MHz): δ = -187.66 (m, 2 F, *p*-F), -172.16 (t, 4 F, *o*-F), - 162.86(dd, 4 F, *m*-F).
**C, H, N-Analyse:** gefunden (berechnet)
C: 47.59%(48.20%), H: 2.75%(3.42%), N. 8.96%(8.88%)

### 10. 1-n-Butyl-2,3-dimethylimidazolium-pentafluorphenylnonafluorbutylsulfonylimid

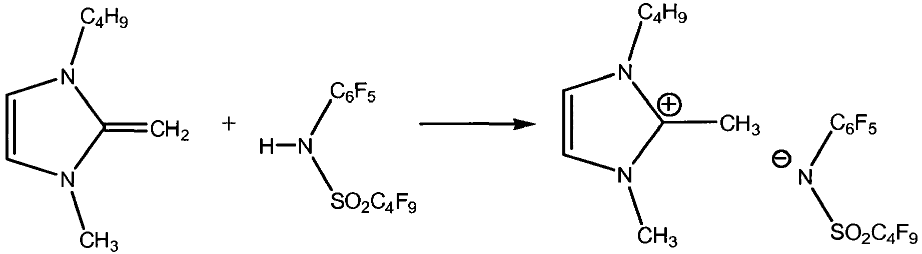

Zu einer auf -78°C gekühlten Lösung von 1.57 g (3.37 mmol) Pentafluorphenylnonafluorbutylsulfonylamin in Diethylether gibt man mittels einer Spritze 0.51 ml (3.37 mmol) 1-n-Butyl-3-methyl-2-methylenimidazolin. Die Reaktionsmischung wird über einen Zeitraum von 5h auf Raumtemperatur erwärmt und 8h bei Raumtemperatur gerührt. Anschließend werden alle flüchtigen Bestandteile im Vakuum entfernt und man erhält das gewünschte Produkt als braunes Öl.

**¹H-NMR** (D₃CCN, 300 MHz): δ = 0.92 (t, 3 H, NCH₂CH₂CH₂C*H*₃), 1.32 (sext, 2 H, NCH₂CH₂C*H*₂CH₃), 1.72 (quint, 2 H, NCH₂C*H*₂CH₂CH₃), 2.50 (s, 3 H, NC*H*₃), 3.69 (s, 3 H, CC*H*₃), 4.02 (t, 2 H, NC*H*₂CH₂CH₂CH₃), 7.25 (s, 1 H, BuNC*H*), 7.27 (s, 1 H, MeNC*H*).
**¹³C-NMR** (D₃CCN, 75 MHz): δ = 8.78 (NCH₂CH₂CH₂*C*H₃), 12.42 (NCH₂CH₂*C*H₂CH₃), 18.84 (*C*CH₃), 31.03 (N*C*H₃), 34.45 (NCH₂*C*H₂CH₂CH₃), 47.74 (NCH₂CH₂CH₂CH₃), 120.53 (BuNCH), 121.99 (MeNCH), 144.80 (NCN), 118.83 - 123.55 (m, C(Alk^{F})). 133.86 - 144.92 (m, C(Ar^{F})).
**¹⁹F-NMR** (D₃CCN, 188 MHz): δ = -165.17 (m, 1 F, *p*-F), -163.79 (t, 2 F, *m*-F), - 146.56 (d, 4 F, *m*-F), -121.82 (2 F,CF₂SO₂), -116.74 (2 F, C*F*₂CF₂SO₂), -109.97 (2 F, C*F*₂CF₃), -76.88 (3 F, CF₃).

### 11. Pentafluorphenyl-nonafluorbutylsulfonylimin

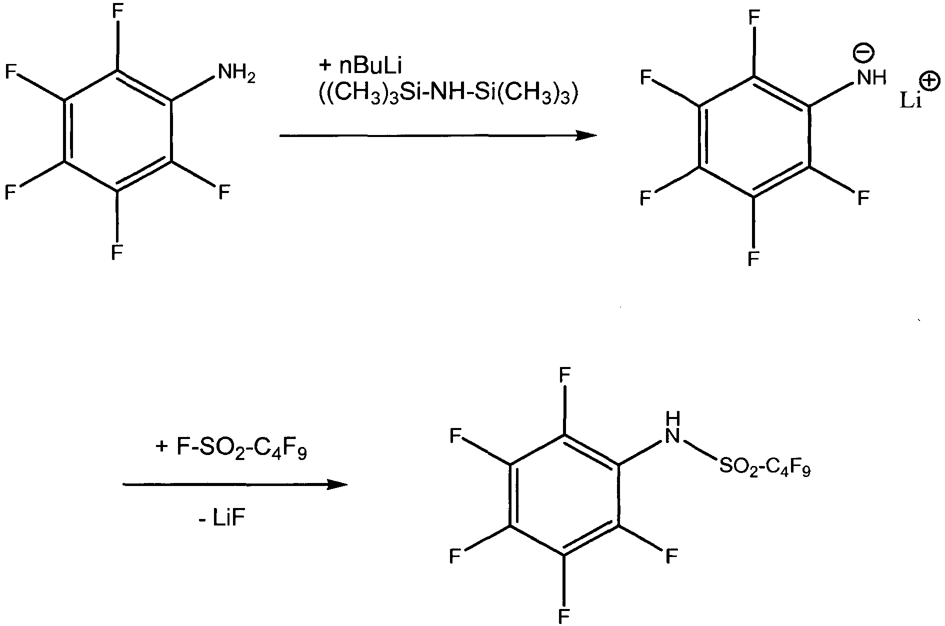

Zu einer auf -78°C gekühlten Lösung von 22.27 ml (106.78 mmol) Hexamethyldisilazan in 50 ml THF gibt man zunächst 66.74 ml einer 1.6-molaren Lösung von n-Butyllithium in Hexan (entspricht 106.78 mmol). Die Lösung wird auf Raumtemperatur erwärmt und für 1 h bei dieser Temperatur gerührt. Anschließend kühlt man wieder auf -78°C und fügt eine Lösung von 7.82 g (42.71 mmol) Pentafluoranilin in 100 ml THF hinzu. Die Reaktionsmischung wird auf eine Temperatur von 0°C gebracht und 2 h bei dieser Temperatur gehalten. Es wird erneut auf -78°C abgekühlt und mittels einer Spritze 7.67 ml Nonafluorbutylsulfonylfluorid hinzugegeben. Die Reaktionsmischung wird über einen Zeitraum von 8 h auf Raumtemperatur gebracht und anschließend 2 Tage bei Raumtemperatur gerührt. Anschließend versetzt man mit 200 ml Wasser und bringt die wässrige Phase mittels halbkonzentrierter HCl auf einen pH-Wert von 6. Die Mischung wird mit dreimal 100 ml Diethylether extrahiert, die vereinigten organischen Phasen über Natriumsulfat getrocknet und anschließend alle flüchtigen Verbindungen am Rotationsverdampfer entfernt. Das erhaltene Rohprodukt wird in siedendem Toluol umkristallisiert. Man erhält ein beiges Pulver.

**¹⁹F-NMR** (D₃CCN, 188 MHz): δ = -169.40 (m, 1 F, p-F), -168.53 (t, 2 F, *m*-F), - 151.21 (d, 2 F, *m*-F), -126.62 (2 F,CF₂SO₂), -121.56 (2 F, C*F*₂CF₂SO₂), -114.74 (2 F, C*F*₂CF₃), -81.70 (3 F, CF₃).

### 12. 1-n-Butyl-3-methylimidazolium-pentafluorphenylnonafluorbutylsulfonylimid

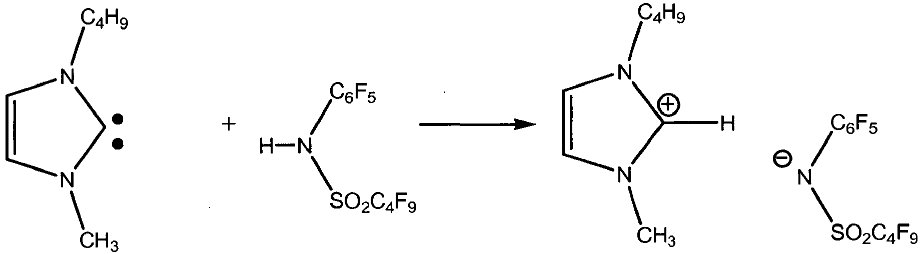

Zu einer auf -78°C gekühlten Lösung von 1.34 g (2.877 mmol) Pentafluorphenylnonafluorbutylsulfonylamin in Diethylether gibt man mittels einer Spritze 0.51 ml (3.37 mmol) 1-n-Butyl-3-methyl-imidazol-2-yliden. Die Reaktionsmischung wird über einen Zeitraum von 5h auf Raumtemperatur erwärmt und 8h bei Raumtemperatur gerührt. Anschließend werden alle flüchtigen Bestandteile im Vakuum entfernt und man erhält das gewünschte Produkt als braunes Öl.

**¹H-NMR** (D₃CCN, 300 MHz): δ = 0.93 (t, 3 H, NCH₂CH₂CH₂C*H*₃), 1.31 (sext, 2 H, NCH₂CH₂C*H*₂CH₃), 1.78 (quint, 2 H, NCH₂C*H*₂CH₂CH₃), 3.81 (s, 3 H, NC*H*₃), 4.11 (t, 2 H, NC*H*₂CH₂CH₂CH₃), 7.33 (s, 1 H, BuNC*H*), 7.36 (s, 1 H, MeNC*H*), 8.44 (s, 1 H, N₂C*H*).
**¹³C**-**NMR** (D₃CCN, 75 MHz): δ = 13.63 (NCH₂CH₂CH₂*C*H₃), 19.98 (NCH₂CH₂*C*H₂CH₃), 32.61 (NCH₂*C*H₂CH₂CH₃), 36.86 (N*C*H₃), 50.32 (N*C*H₂CH₂CH₂CH₃), 123.32 (BuN*C*H), 124.68 (MeN*C*H), 137.00 (N*C*N).
**¹⁹F-NMR** (D₃CCN, 188 MHz): δ = -165.28 (m, 1 F, *p*-F), -163.82 (t, 2 F, *m*-F), - 146.64 (d, 2 F, *m*-F), -121.76 (2 F,CF₂SO₂), -116.72 (2 F, C*F*₂CF₂SO₂), -109.99 (2 F, C*F*₂CF₃), -76.85 (3 F, CF₃).

### 13. 1-n-Butyl-3-methylimidazolium-decafluordiphenylimid (nicht erfindungsgemäß)

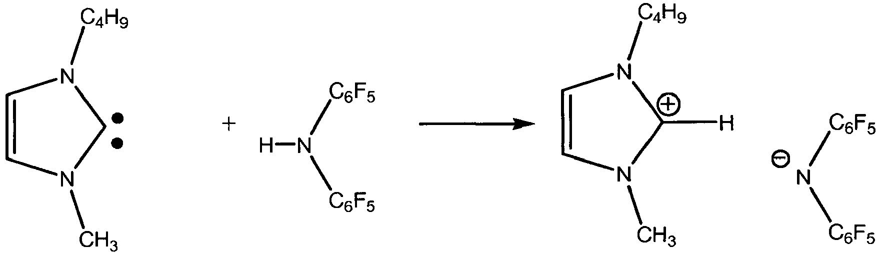

Zu einer auf -78°C gekühlten Lösung von 0.88 g (2.52 mmol) Decafluordiphenylamin in Diethylether gibt man mittels einer Spritze 0.31 ml (2.52 mmol) 1-n-Butyl-3-methyl-imidazol-2-yliden. Die Reaktionsmischung wird über einen Zeitraum von 5h auf Raumtemperatur erwärmt und 8h bei Raumtemperatur gerührt. Anschließend werden alle flüchtigen Bestandteile im Vakuum entfernt und man erhält das gewünschte Produkt als braunes Öl.

**¹H-NMR** (D₃CCN, 300 MHz): δ = 0.88 (t, 3 H, NCH₂CH₂CH₂C*H*₃), 1.27 (sext, 2 H, NCH₂CH₂C*H*₂CH₃), 1.75 (quint, 2 H, NCH₂C*H*₂CH₂CH₃), 3.80 (s, 3 H, NC*H*₃), 4.09 (t, 2 H, NC*H*₂CH₂CH₂CH₃), 7.34 (s, 1 H, BuNC*H*), 7.39 (s, 1 H, MeNC*H*), 8.87 (s, 1 H, N₂C*H*).
**¹³C**-**NMR** (D₃CCN, 75 MHz): δ = 13.63 (NCH₂CH₂CH₂*C*H₃), 20.07 (NCH₂CH₂*C*H₂CH₃), 32.69 (NCH₂*C*H₂CH₂CH₃), 36.78 (N*C*H₃), 50.38 (N*C*H₂CH₂CH₂CH₃), 123.34 (BuN*C*H), 124.69 (MeN*C*H), 137.60 (N*C*N), 131.92 - 143.18 (m, C(Ar^{F})).
**¹⁹F-NMR** (D₃CCN, 282 MHz): δ = -186.18 ppm (t, 1 F, *p*-F), -171.60 ppm (2 F, *m*-F), -162.22 ppm (d, 1 F, o-F).

### 14. Darstellung von nBu₄N(N(C₆F₅)₂] (nicht erfindungsgemäß)

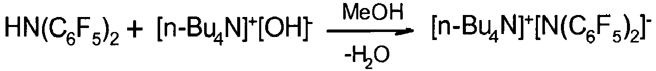

Zu einer Lösung von 0.40 g (1.15 mmol) DFDPA-H in 5 ml Methanol werden 1.15 ml der Tetra-n-butylammoniumhydroxid - Lösung (Aldrich; 1 M, 1.15 mmol) in Methanol bei RT zugegeben. Das Reaktionsgemisch wird 2 h bei RT gerührt. Das Lösungsmittel wird im Vakuum entfernt, der Rückstand aus Diethylether umkristallisiert und im Vakuum getrocknet. Weiße Nadeln. Ausbeute 0.55 g (81%).
Smp. 97.6°C
**C₂₈H₃₆F₁₀N₂O** (590.58 g/mol). Ber. (gef.) C 56.94 (56.92); N 4.74 (5.11); H 6.14 (6.67).
**¹H-NMR** (400.0 MHz, THF-d₈): δ = 0.95 (t, 3H, CH₃, ³J_{H-H} =7.36 Hz), 1.30 - 1.40 (m, 2H, CH₂), 1.62 - 1.70 (m, 2H, CH₂), 3.21 - 3.25 (m, 2H, CH₂) ppm.
**¹³C**-**NMR** (125.7 MHz, THF-d₈): δ = 13.7 (s, CH₃), 20.4 (s, CH₂), 24.4 (s, CH₂), 59.2 (s, NCH₂), 129.2 (dm, ¹J_{C-F} =231.3 Hz, C₆F₅), 134.4 (t, ²J_{C-F} =12.1 Hz, C₆F₅), 138.8 (dm, ¹J_{C-F} =241.1 Hz, C₆F₅), 141.3 (dm, ¹J_{C-F}=233.8 Hz, C₆F₅) ppm.
**¹⁹F-NMR** (188.2 MHz, THF-d₈)_{:} δ = -183.6 (m, 1F, CFpₐᵣₐ), -167.5 (t, ³J_{F-F} = 20 Hz, 2F, CFₘₑₜₐ), -156.8 (m, 2F, CFₒᵣₜₕₒ) ppm.
**IR** (Nujol): *ṽ* = 2726 w, 2602 w, 1635 w, 1510 m, 1480 s, 1465 s, 1379 m, 1307 m, 1261 w, 1195 w, 1024 s, 995 s, 966 m, 706 m, 644 m, 559 m, 424 w cm⁻¹.

### Kristallstrukturanalyse von Bu₄N [N(C₆F₅)₂]

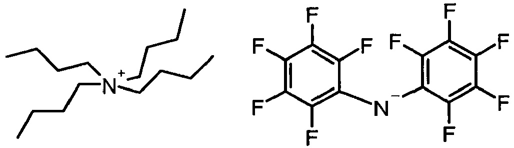

| | | |
|---|---|---|
| Form, Farbe | Nadeln, farblos | |
| Kristallgröße | 0.30 x 0.10 x 0.06 mm³ | |
| Kristallsystem | Triklin | |
| Raumgruppe | P 1̅ | Z = 2 |
| Elementarzelle | a = 8.6597(12) A | α = 75.023(11)°. |
| | b = 12.0615(16) A | β = 88.185(11)°. |
| | c = 14.383(2) A | γ = 74.643(10)°. |
| Volumen | 1398.4(3) A3 | |
| Elementarzellbestimmung | 10121 Reflexionen | |
| Empirische Formel | C₂₈ H₃₆ F₁₀ N₂ | |
| Molekulargewicht | 590.59 | |
| Dichte (berechnet) | 1.403 Mg/m³ | |
| Absorptionskoeffizient | 0.129 mm⁻¹ | |
| F(000) | 616 | |
| Diffraktometertyp | IPDS2 | |
| Wellenlänge | 0.71073 A | |
| Temperatur | 193(2) K | |
| Thetabereich für die Datenerfassung | 1.47 to 26.23°. | |
| Indexbereiche | -10<=h<=10, -14<=k<=14, -17<=I<=17 | |
| Datenerfassungssoftware | STOE Win-Xpose (X-Area) | |
| Zellverfeinerungssoftware | STOE Win-Cell (X-Area) | |
| Datenreduktionssoftware | STOE Win-Integrate (X-Area) | |
| Erfasste Reflexionen | 18043 | |
| Unabhängige Reflexionen | 5590 [R(int) = 0.0544] | |
| Übereinstimmung mit theta = 26.23° | 99.4 % | |
| Beobachtete Reflexe | 3269[I>2sigma(I)] | |
| Für die Feinabstimmung verwendete Reflexe | | 5590 |
| Extin ktionskoeffizient | X = 0.0160(16) | |
| Absorptionskorrektur | None | |
| Max. and min. Transmission | 0.9923 and 0.9625 | |
| Größter Diffraktionspeak und -volumen | 0.189 and -0.172 e.A-3 | |
| Auflösung | Direct methods | |
| Verfeinerung | Full-matrix least-squares on F² | |
| Wasserstoffatome | Berechnete Positionen, | |
| U(H)=1.2(1.5)*Ueq(C) | | |
| Verwendete Programme | SHELXS-97 (Sheldrick, 1997) | |
| | SHELXL-97 (Sheldrick, 1997) | |
| | DIAMOND 2.1, STOE IPDS software | |
| Daten / Einschränkungen / Parameter | 5590/0/366 | |
| Anpassungstest für F² | 0.875 | |
| R Index (alle Daten) | wR2 = 0.0976 | |
| R Index konventionell [I>2sigma(I)] | R1 = 0.0396 | |

### 15. Methyltriphenylphosphonium-decafluordiphenylimid (nicht erfindungsgenäß)

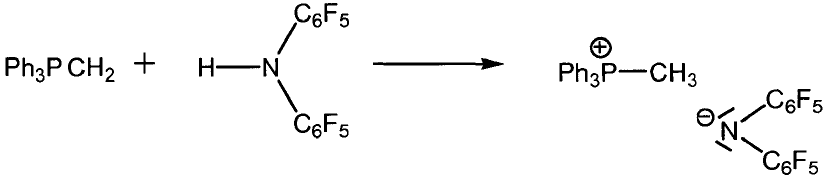

Zu einer Lösung von 1.00 g (2.87 mmol) Decafluordiphenylamin in 20 ml Toluol gibt man bei Raumtemperatur eine Lösung von 0.79 g (2.87 mmol) Triphenylphosphoniummethylid in 10 ml Toluol. Es bildet sich sofort ein farbloser Niederschlag. Die Reaktionsmischung wird noch 1 h bei Raumtemperatur nachgerührt und anschließend über eine Umkehrfritte abfiltriert. Der erhaltene Feststoff wird mit 20 ml Hexan gewaschen und im Vakuum getrocknet. Man erhält das gewünschte Produkt als einen farblosen Feststoff.

**¹H-NMR** (D₃CCN, 200 MHz): δ = 2.81 (d, 2 H, PC*H*₃), 7.60-7.90 (m, 15 H, Ar-*H*).
**¹³C-NMR** (D₃CCN, 50 MHz): δ = 9.28 (d, PCH₃), 120.37 (d, *i*-C), 131.15 (d, *o*-C), 134.24 (d, *m*-C), 136.08 (d, p-C).
**¹⁹F-NMR** (D₃CCN, 188 MHz): δ = -182.48 (t, 2 F, *p*-F), -167.12 (4 F, *m*-F), -157.77 (d, 4 F, *o*-F).
**³¹P-NMR** (D₃CCN, 81 MHz): δ = 31.37 (Ph₃*P*CH₃).

### Abbildungslegenden und Bezugszeichenliste

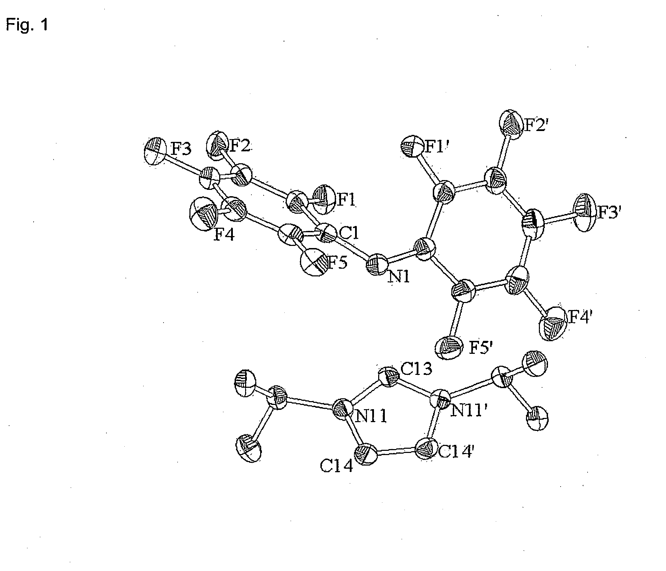

Fig. 1:Strukturmodell von 1,3-Di(isopropyl)imidazolium-decafluordiphenylamid

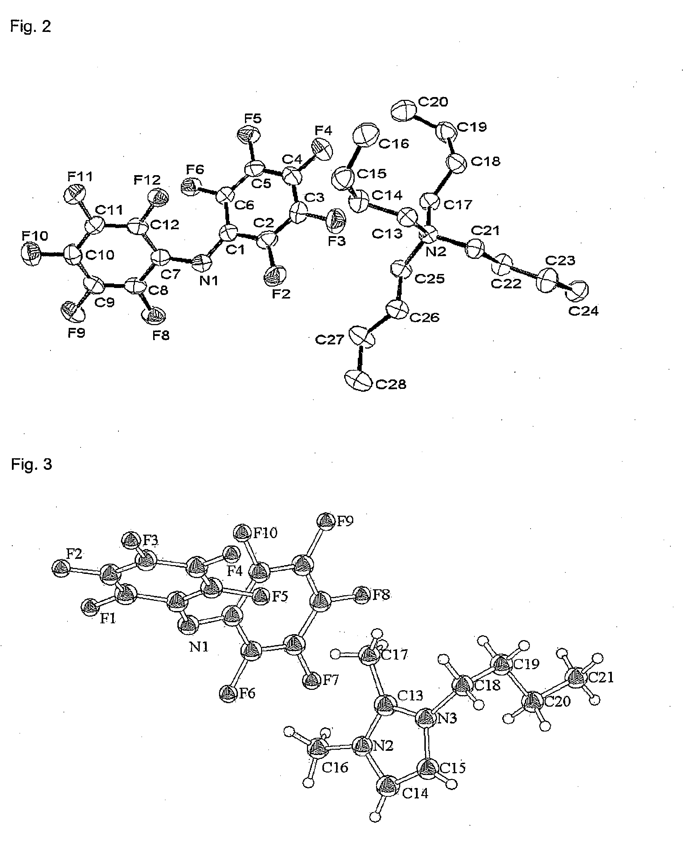

Fig. 2: Strukturmodell von Bu₄N[N(C₆F₅)₂]
Fig. 3: Strukturmodell von 1-n-Butyl-2,3-dimethylimidazolium-decafluorodiphenylamid

## Patentansprüche

1. Salz umfassend
a) ein Anion der allgemeinen Formel 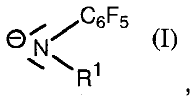 und
b) ein Kation, ausgewählt aus
anorganischen Kationen aus der Gruppe der Alkalikationen und Erdalkalikationen oder
quaternären organischen Kationen,
c) **dadurch gekennzeichnet, dass** bei dem Anion
R¹ = -SO₂-R² darstellt, wobei
R₂ eine verzweigte oder unverzweigte Alkylgruppe mit 1 bis 20 C-Atomen oder eine Aryl- oder Benzylgruppe darstellt und diese Alkyl-, Benzyl- oder Arylgruppe partiell oder vollständig fluoriert ist.

2. Salz gemäß Anspruch 1, **dadurch gekennzeichnet, dass** das Kation ausgewählt der Gruppe quaternären Ammoniumionen, Phosphoniumionen, Guanidiniumionen, Imidazoliumionen, Imidazolidiniumionen, Benzimidazoliumionen und N-Organo-Pyridiniumionen.

3. Salz gemäß Anspruch 2, **dadurch gekennzeichnet, dass** das Kation ein Ammoniumion der allgemeinen Formel
[NR³R⁴R⁵R⁶]⁺ **(II)** ist,
worin
R³, R⁴ und R⁵ unabhängig voneinander eine lineare oder verzweigte Alkylgruppe mit 1 bis 20 C-Atomen oder eine Arylgruppe oder eine Benzylgruppe darstellen und R⁶ eine lineare oder verzweigte Alkylgruppe mit 1 bis 20 C-Atomen darstellt.

4. Salz gemäß Anspruch 2, **dadurch gekennzeichnet, dass** das Kation ein Phosphoniumion der allgemeinen Formel
[PR³R⁴R⁵R⁷]⁺ **(III)** ist,
worin R³, R⁴ und R⁵ die oben aufgeführten Bedeutungen haben und
R⁷ eine lineare oder verzweigte Alkylgruppe mit 1 bis 20 C-Atomen oder eine Arylgruppe oder eine Benzylgruppe darstellt.

5. Salz gemäß Anspruch 2, **dadurch gekennzeichnet, dass** das Kation ein Imidazoliumion der allgemeinen Formel 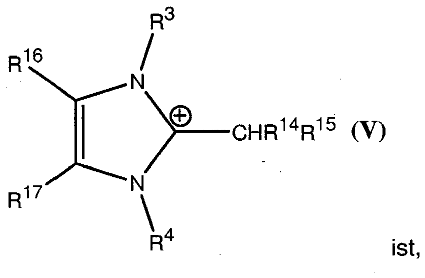 worin
R³ und R⁴ wie oben definiert sind und
R¹⁴, R¹⁵, R¹⁶ und R¹⁷ unabhängig voneinander jeweils für ein H-Atom, eine verzweigte oder unverzweigte Alkylgruppe mit 1 bis 20 C-Atomen, eine Aryl- oder eine Benzylgruppe stehen.

6. Salz gemäß Anspruch 2, **dadurch gekennzeichnet, dass** das Kation ein Imidazolidiniumion der allgemeinen Formel 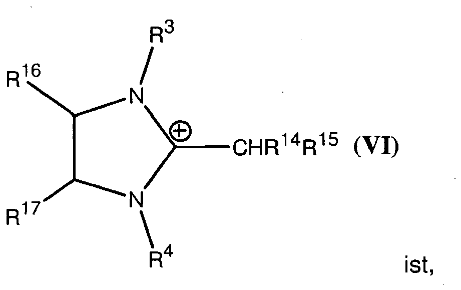 worin R³, R⁴, R¹⁴, R¹⁵, R¹⁶ und R¹⁷ wie oben definiert sind.

7. Salz gemäß Anspruch 2, **dadurch gekennzeichnet, dass** das Kation ein Benzimidazolidiniumkationen der allgemeinen Formel 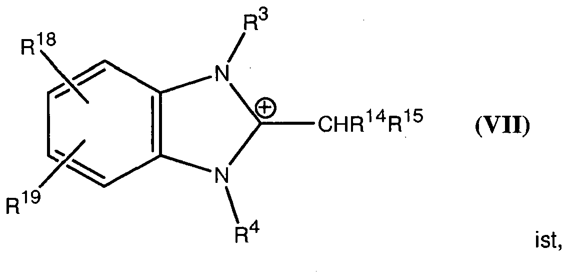 worin R³, R⁴, R¹⁴ und R¹⁵ wie oben definiert sind und
R¹⁸ und R¹⁹ unabhängig voneinander für ein H-Atom, F, Cl, eine lineare oder verzweigte Alkylgruppe mit 1 bis 20 C-Atomen, eine Aryl- oder eine Benzylgruppe stehen.

8. Verfahren zur Herstellung von Salzen gemäß Anspruch 1, **dadurch gekennzeichnet, dass** die korrespondierende Säure 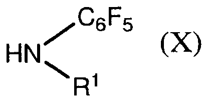 des Anions gemäß Formel (I) mit der korrespondierenden Verbindung eines Kations in einem organischen Lösungsmittel umgesetzt wird.

9. Verwendung von Salzen gemäß einem der Ansprüche 1 bis 7 als Bestandteil einer ionischen Flüssigkeit für Elektrolytsysteme in Batterien, galvanischen Elementen und Akkumulatoren.

10. Verwendung eines Salzes gemäß Anspruch 9 in einem Lithiumionenakku.

11. Verwendung eines Salzes gemäß einem der Ansprüche 1 bis 7 als Lösungsmittel für die Mehrphasenkatalyse.

12. Verwendung eines Salzes gemäß einem der Ansprüche 1 bis 7 als nichtwässriger Elektrolyt.

13. Verwendung eines Salzes gemäß einem der Ansprüche 1 bis 7 als mobile und/oder stationäre Phase in der Chromatographie.

14. Verfahren zur Herstellung ionischer Flüssigkeiten nach Anspruch 8, **dadurch gekennzeichnet, dass** ein Keten-N,N-diacetal mit der korrespondierenden N-H-Säure gemäß Formel (X) in einem organischen Lösungsmittel umgesetzt wird.

15. Verfahren zur Herstellung ionischer Flüssigkeiten nach Anspruch 8, **dadurch gekennzeichnet, dass** ein Alkyl-alkyliden-phosphoran oder Aryl-alkyliden-phosphoran mit einer Säure der allgemeinen Formel 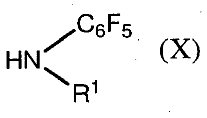 worin
R¹ gemäß Anspruch 1 definiert ist,
in einem organischen Lösungsmittel umgesetzt wird.

## Claims

1. Salt comprising
a) an anion of the general formula 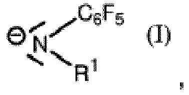 and
b) a cation selected from
inorganic cations from the group of alkali cations and
earth alkali cations or
quaternary organic cations
c) **characterised in that** the in the anion
R¹ denotes -SO₂-R² wherein
R₂ is a branched or unbranched alkyl group with 1 to 20 C atoms or an aryl or benzyl group and this alkyl, benzyl or aryl group is partially or completely fluoridised.

2. Salt according to claim 1 **characterised in that** the cation is selected from the group quaternary ammonium ions, phosphonium ions, guanidinium ions, imidazolium ions, imidazolidinium ions, benzimidazolium ions and N-organo-pyridinium ions.

3. Salt according to claim 2 **characterised in that** the cation is an ammonium ion of the general formula
[NR³R⁴R⁵R⁶]⁺ (II)
wherein
R³, R⁴ and R⁵ independently from one another denote a linear or branched alkyl group with 1 to 20 C atoms or an aryl group or a benzyl group, and R⁶ denotes a linear or branched alkyl group with 1 to 20 C atoms.

4. Salt according to claim 2 **characterised in that** the cation is a phosphonium ion of the general formula
[PR³R⁴R⁵R⁷]⁺ (III)
wherein R², R⁴, R⁵ have the meanings set out above and R⁷ is a linear or branched alkyl group with 1 to 20 C atoms or an aryl group or a benzyl group.

5. Salt according to claim 2 **characterised in that** the cation is an imidazolium ion of the general formula 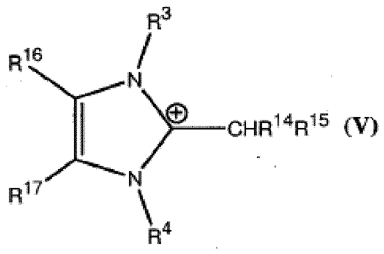 wherein R³ and R⁴ are as defined above and
R¹⁴, R¹⁵, R¹⁶ and R¹⁷ independently of each other stand for an H atom, a branched or unbranched alkyl group with 1 to 20 atoms, an aryl or a benzyl group.

6. Salt according to claim 2 **characterised in that** the cation is an imidazolidium ion of the general formula 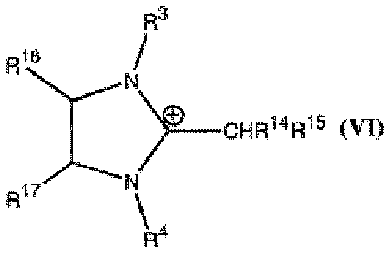 wherein R³, R⁴. R¹⁴, R¹⁵, R¹⁶ and R¹⁷ are as defined above.

7. Salt according to claim 2 **characterised in that** the cation is a benzimidazolidinium cation of the general formula 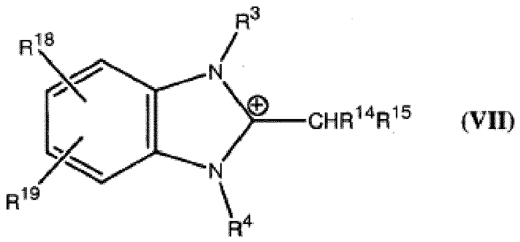 wherein R³, R⁴, R¹⁴ and R¹⁵ are as defined above and
R¹⁸ and R¹⁹ independently of each other stand for an H atom, F, Cl, a linear or branched alkyl group with 1 to 20 atoms, an aryl or a benzyl group.

8. Method of producing salts according to claim 1 **characterised in that** the corresponding acid 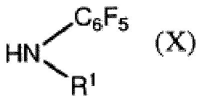 of the anion in accordance with formula (I) is converted with the corresponding compound of a cation in an organic solvent.

9. Use of salts according to any one of claims 1 to 7 as a constituent of an ionic fluid of electrolyte systems in batteries, galvanic elements and rechargeable batteries.

10. Use of a salt according to claim 9 in a lithium ion rechargeable battery.

11. Use of a salt according to any one of claims 1 to 7 as a solvent for multiphase catalysis.

12. Use of a salt according to any one of claims 1 to 7 as a nonaqueous electrolyte.

13. Use of a salt according to any one of claims 1 to 7 as a mobile or stationary phase in chromatography.

14. Method of producing ionic fluids according to claim 8 **characterised in that** a ketene-N,N-diacetal is converted with the corresponding N-H acid in accordance with formula (X) in an organic solvent.

15. Method of producing ionic fluids according to claim 8 **characterised in that** an alkyl-alkylidene phosphorane or aryl-alkylidene phosphorane is converted in an organic solvent with an acid of the general formula 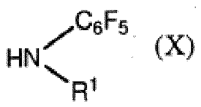 wherein
R¹ is defined in accordance with claim 1.

## Revendications

1. Sel comprenant
a) un anion de formule générale 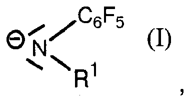 et
b) un cation, choisi parmi
- des cations anorganiques du groupe des cations alcalins et des cations alcalino-terreux ou
des cations quaternaires organiques,
c) **caractérisé en ce que**, pour l'anion,
R^{l}=-SO₂-R²,
R² représentant un groupe alkyle ramifié ou non ramifié avec 1 à 20 atomes de C ou un groupe aryle ou benzyle et ce groupe alkyle, benzyle ou aryle étant partiellement ou complètement fluoré.

2. Sel selon la revendication 1, **caractérisé en ce que** le cation est choisi dans le groupe des ions quaternaires ammonium, phosphonium, guanidinium, imidazolium, imidazolidinium, benzimidazolium et N-organo-pyridinium.

3. Sel selon la revendication 2, **caractérisé en ce que** le cation est un ion ammonium de formule générale
[NR³R⁴R⁵R⁶]⁺ (II)
R³, R⁴ et R⁵ représentant, indépendamment les uns des autres, un groupe alkyle linéaire ou ramifié avec 1 à 20 atomes de C ou un groupe aryle ou un groupe benzyle et R⁶ représentant un groupe alkyle linéaire ou ramifié avec 1 à 20 atomes de C.

4. Sel selon la revendication 2, **caractérisé en ce que** le cation est un ion phosphonium de formule générale
[PR³R⁴R⁵R⁷]⁺ (III)
R³, R⁴ et R⁵ ayant les significations données ci-dessus et R⁷ représentant un groupe alkyle linéaire ou ramifié avec 1 à 20 atomes de C ou un groupe aryle ou un groupe benzyle.

5. Sel selon la revendication 2, **caractérisé en ce que** le cation est un ion imidazolium de formule générale 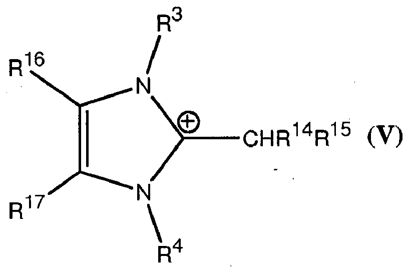 R³ et R⁴ étant définis comme ci-dessus et R¹⁴, R¹⁵, R¹⁶ et R¹⁷ représentant indépendamment les uns des autres, à chaque fois, un atome H, un groupe alkyle ramifié ou non ramifié avec 1 à 20 atomes de C, un groupe aryle ou un groupe benzyle.

6. Sel selon la revendication 2, **caractérisé en ce que** le cation est un ion imidazolidinium de formule générale 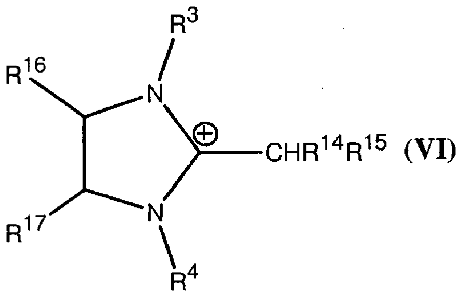 R³ R⁴, R¹⁴ R¹⁵ R¹⁶ et R¹⁷ étant définis comme ci-dessus.

7. Sel selon la revendication 2, **caractérisé en ce que** le cation est un ion benzimidazolidinium de formule générale 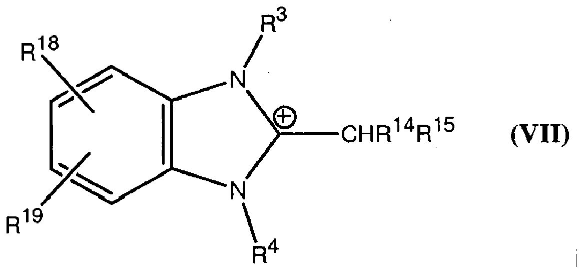 R³, R⁴, R¹⁴ et R¹⁵ étant définis comme ci-dessus et R¹⁸ et R¹⁹ représentant indépendamment les uns des autres un atome H, F, Cl, un groupe alkyle linéaire ou ramifié avec 1 à 20 atomes de C, un groupe aryle ou un groupe benzyle.

8. Procédé de fabrication de sels selon la revendication 1, **caractérisé en ce que** l'acide correspondant 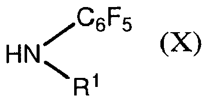 de l'anion selon la formule (I) est transformé avec la liaison correspondante d'un cation en un solvant organique.

9. Utilisation de sels selon l'une des revendications 1 à 7 comme constituant d'un liquide ionique pour des systèmes électrolytiques dans des batteries, des éléments galvaniques et des accumulateurs.

10. Utilisation d'un sel selon la revendication 9 dans une batterie lithium-ions.

11. Utilisation d'un sel selon l'une des revendications 1 à 7 comme solvant pour la catalyse multiphase.

12. Utilisation d'un sel selon l'une des revendications 1 à 7 comme électrolyte non aqueux.

13. Utilisation d'un sel selon l'une des revendications 1 à 7 comme phase mobile et/ou stationnaire en chromatographie.

14. Procédé de fabrication de liquides ioniques selon la revendication 8, **caractérisé en ce qu'**un cétène-N,N-diacétal est transformé avec l'acide N-H selon la formule (X) correspondant en un solvant organique.

15. Procédé de fabrication de liquides ioniques selon la revendication 8, **caractérisé en ce qu'**un phosphorane d'alkyle-alkylide ou un phosphorane d'aryle-alkylide est transformé avec un acide de formule générale 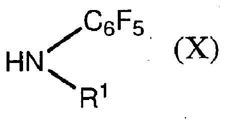 R¹ étant défini selon la revendication 1, en un solvant organique.
